(19) Europäisches Patentamt
European Patent Office
Office européen des brevets

(11) Veröffentlichungsnummer: **0 057 365**
**B1**

(12) **EUROPÄISCHE PATENTSCHRIFT**

(45) Veröffentlichungstag der Patentschrift: **11.07.84**

(51) Int. Cl.³: **C 07 D 233/58**, C 07 D 233/56, C 07 D 249/08, A 01 N 43/50, A 01 N 43/64

(21) Anmeldenummer: **82100308.4**

(22) Anmeldetag: **18.01.82**

(54) **Vinylazole, Verfahren zu ihrer Herstellung und ihre Verwendung bei der Bekämpfung von Pilzen.**

(30) Priorität: **30.01.81 DE 3103068**

(43) Veröffentlichungstag der Anmeldung: **11.08.82 Patentblatt 82/32**

(45) Bekanntmachung des Hinweises auf die Patenterteilung: **11.07.84 Patentblatt 84/28**

(84) Benannte Vertragsstaaten: **AT BE CH DE FR GB IT LI NL SE**

(56) Entgegenhaltungen:
**DE - A - 1 542 690**
**DE - A - 2 645 617**
**DE - A - 2 903 653**
**FR - A - 2 436 780**

**J. Heterocyclic Chem., 15, 1543-1545 (1978)**

(73) Patentinhaber: **BASF Aktiengesellschaft, Carl-Bosch-Strasse 38, D-6700 Ludwigshafen (DE)**

(72) Erfinder: **Meyer, Norbert, Dr., Stahlbuehlring 155A, D-6802 Ladenburg (DE)**
Erfinder: **Zeeh, Bernd, Dr., Thorwaldsenstrasse 5, D-6700 Ludwigshafen (DE)**
Erfinder: **Buschmann, Ernst, Dr., Georg-Ludwig-Krebs-Strasse 10, D-6700 Ludwigshafen (DE)**
Erfinder: **Pommer, Ernst-Heinrich, Dr., Berliner Platz 7, D-6703 Limburgerhof (DE)**

**EP 0 057 365 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Vinylazole, Verfahren zu ihrer Herstellung, Fungizide, die diese Verbindungen als Wirkstoffe enthalten, sowie ein Verfahren zur Bekämpfung von Pilzen mit diesen Verbindungen.

Es ist bekannt, dass Vinylazole fungizid wirksam sind DE-OS 26 45 617).

Es wurde gefunden, dass Vinylazole der Formel

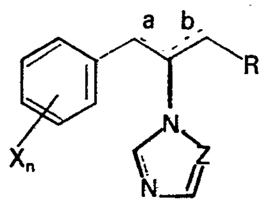

(I)

in der

R Alkyl mit 1 bis 7 Kohlenstoffatomen oder gegebenenfalls durch Halogen substituiertes Phenyl,

X Wasserstoff, Halogen, Alkyl mit 1 bis 4 Kohlenstoffatomen oder Phenyl,

Z N oder CH und

n eine ganze Zahl von 1 bis 5 bedeuten und die Doppelbindung entweder die Position a oder b einnimmt, sowie deren pflanzenverträgliche Säureadditionssalze und Metallkomplexe bekannte Vinylazole in ihrer fungiziden Wirksamkeit übertreffen.

Die Verbindungen der Formel I können als E/Z-Isomere vorliegen. Unter der Bezeichnung Vinylazole sind dementsprechend sowohl die reinen Isomeren als auch ihre Gemische zu verstehen.

Bei der Herstellung der Vinylazole der Formel I können Gemische aus Verbindungen entstehen, die hinsichtlich R, X, Z und n identisch sind, die sich aber durch die Position der Doppelbindung unterscheiden. Diese Gemische werden von Formel I umfasst; in der Beschreibung sind sie durch die Angabe a/b zur Position der Doppelbindung charakterisiert.

In der Formel I bedeutet R einen unverzweigten oder verzweigten Alkylrest mit 1 bis 7 Kohlenstoffatomen, wie Methyl, Ethyl, Isopropyl, n-Propyl, tert. Butyl, n-Butyl, n-Pentyl, n-Hexyl, n-Heptyl oder einen Phenylrest, der durch Halogen, wie Chlor oder Brom, einfach oder mehrfach substituiert sein kann, wie 4-Chlorphenyl, 2,4-Dichlorphenyl, 4-Bromphenyl.

X in Formel I bedeutet ausser Wasserstoff oder Phenyl Halogen, wie Chlor, Brom, Jod, oder einen unverzweigten oder verzweigten Alkylrest mit 1 bis 4 Kohlenstoffatomen, wie Methyl, Ethyl, n-Propyl, sec-Butyl, Isobutyl, tert.-Butyl.

Bevorzugt sind Verbindungen der Formel I, bei denen R einen Alkylrest bedeutet und die Doppelbindung die Position a oder b einnimmt. $X_n$ bedeutet in diesen bevorzugten Verbindungen beispielsweise 2-Chlor, 4-Brom, 24-Dichlor, 4-Methyl, 4-Phenyl.

Man erhält die Vinylazole der Formel I durch Eliminierung von HY aus Verbindungen der Formeln

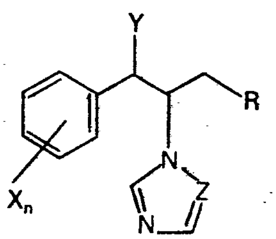

(II) oder

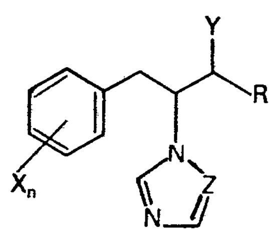

(III)

in denen

R, X, Z und n die oben angegebenen Bedeutungen haben und Y eine zugängliche Abgangsgruppe darstellt.

Als Abgangsgruppen für Y kommen Hydroxy, Tosylat, Mesylat, Halogenid, Acetat, Trifluoracetat, Xanthogenat, in Betracht.

Ist Y eine Hydroxygruppe, so erhält man die Vinylazole der Formel I durch Eliminierung von Wasser in Gegenwart eines sauren Dehydratisierungskatalysators aus den entsprechenden α-Azolylalkoholen der Formeln II bzw. III. Geeignete Katalysatoren sind Protonsäuren, Lewis-Säuren, Säureanhydride oder Säurechloride, beispielsweise Schwefel-, Phosphor- oder Ameisensäure, p-Toluolsulfonsäure, Bortrifluorid, Diphosphorpentoxid, Acetanhydrid, Phosphoroxichlorid, Thionylchlorid.

Die Menge an saurem Katalysator beträgt 10 bis 200 Gew.-% des α-Azolylalkohols.

Die Wasserabspaltung wird bei einer Temperatur im Bereich zwischen 70 und 180 °C, vorzugsweise zwischen 80 und 120 °C, durchgeführt. Sie kann in lösungsmittelfreiem Medium durchgeführt werden. Vorzugsweise arbeitet man jedoch in Gegenwart eines Lösungsmittels.

Hierfür geeignete Lösungsmittel sind Toluol, Xylol, Pyridin, Eisessig, Acetonitril.

Die Hydroxyfunktion kann auch in eine leichter abspaltbare Gruppe, z.B. in Tosylat, Halogenid oder Acetat, überführt werden. Diese Abgangsgruppe lässt sich dann in Gegenwart eines basischen Katalysators, beispielsweise eines Alkoholats, eines tertiären Amins, eines Alkalicarbonats, wie Natriummethylat, Kalium-tert.-butylat, Pyridin, Kaliumcarbonat, oder in Gegenwart von Natriumsulfid bei einer Temperatur zwischen 20 und 100 °C abspalten.

Man verwendet dabei 1 bis 2,5 Mole an basischem Katalysator pro Mol Verbindung der Formel II oder III.

Die Eliminierungsreaktion wird vorzugsweise in Anwesenheit eines Lösungsmittels durchgeführt. Geeignete Lösungsmittel sind aliphatische Alkohole, wie Ethanol, n-Butanol, tert.-Butanol, Octanol, chlorierte Kohlenwasserstoffe, wie Chloroform, Dichlormethan, N,N-Dialkylsäureamide, wie Dimethylformamid, sowie Dimethylsulfoxid, Pyridin, Toluol, Tetranydrofuran, Wasser. Auch Gemische dieser Lösungsmittel können verwendet werden.

Ebenso kann die Hydroxyfunktion auch verestert, d.h. in eine Acetat- oder Xanthogenatgruppe überführt werden, die dann thermisch nach bekannten Methoden eliminiert werden kann (Houben-Weyl, Methoden der organischen Chemie Bd. 5/1b, S. 109 ff, Georg Thieme-Verlag, Stuttgart, 1972).

Die Vinylazole der Formel I können in an sich üblicher Weise in für Pflanzen verträgliche Salze oder Metallkomplexe überführt werden. Zur Salzbildung eignen sich Mineralsäuren, wie Salzsäure, Bromwasserstoffsäure, Schwefelsäure, Salpetersäure, Phosphorsäure, und organische Säuren wie Essigsäure und Dodecylbenzolsulfonsäure. Die fungizide Wirksamkeit der Salze der Vinylazole beruht auf dem Kation so dass das Anion beliebig aus der Vielzahl der pflanzenphysiologisch vertäglichen Anionen ausgewählt werden kann.

Metallkomplexe können durch Anlagerung der Vinylazole an die Kationen von Metallsalzen gebildet werden. Hierfür eignen sich insbesondere Kupfer-, Zink-, Eisen-, Mangan-, und Nickelsalze wie Kupfer(II)-chlorid-, Kupfer(II)-sulfat, Kupfer (II)-nitrat, Zink(II)-chlorid, Eisen(III)-chlorid, Mangan(II)-chlorid, Nickel(II)-bromid.

Die als Ausgangsstoffe verwendeten Verbindungen der Formeln II und III, wobei Y Hydroxy bedeutet, lassen sich nach bekannten Verfahren aus α-Azolylketonen herstellen (DE-OS 27 34 426).

Die folgenden Beispiele erläutern die Herstellung der neuen Verbindungen.

Beispiel 1

1-(2,4-Dichlorphenyl)-4,4-dimethyl-2-[1-(1,2,4-triazolyl)]-1-penten

49,2 g 1-(2,4-Dichlorphenyl)-4,4-dimethyl-2-[1-(1,2,4-triazolyl)]-3-pentanol (als Diastereomerengemisch) werden in 500 ml absolutem Tetrahydrofuran gelöst und unter Rühren bei Raumtemperatur mit 6,0 g 80%iger Natriumhydriddispersion versetzt. Nach achtstündigem Rühren bei 40 bis 50 °C wird auf Raumtemperatur abgekühlt. Dann tropft man eine Lösung von 28,2 g Toluol-4-sulfonylchlorid in 60 ml absolutem Tetrahydrofuran zu, rührt weitere 12 Stunden bei Raumtemperatur, hydrolysiert mit Wasser, extrahiert mehrfach mit 500 ml Methylenchlorid, trocknet die organische Phase anschliessend über Natriumsulfat und engt ein. Durch fraktionierte Kristallisation aus Essigester erhält man 42,5 g der diastereomeren Tosylate, die in 500 ml Dimethylsulfoxid gelöst und mit 54 g gemahlenem Natriumsulfid versetzt werden. Nach einstündigem Rühren bei Raumtemperatur gibt man 1 l Wasser zu, extrahiert zweimal mit je 1 l Diethylether, trocknet die organische Phase über Natriumsulfat und engt ein. Der ölige Rückstand wird in wenig Ethanol aufgenommen und gekühlt; dabei kristallisieren 4,5 g 1-(2,4-Dichlorphenyl)-4,4-dimethyl-2-[1-(1,2,4-triazolyl)]-1-penten vom Fp. 133 °C aus (Isomer A). Durch Einengen der Mutterlauge erhält man 14,0 g 1-(2,4-Dichlorphenyl)-4,4 -dimethyl-2-[1-(1,2,4-triazolyl)]-1-penten vom Sdp. 132 bis 138 °C /0,05 mbar als Öl. (Isomer B)

Beispiel 2

1-(2,4-Dichlorphenyl)-2-[1-(1,2,4,-triazolyl]-2-penten

Zu einer Lösung von 107 g 1-(2,4-Dichlorphenyl)-2[1-(1,2,4-triazolyl)]-1-pentanol in 500 ml Chloroform tropft man bei Raumtemperatur 42,9 g Thionylchlorid, lässt 12 Stunden nachrühren und erhitzt anschliessend 4 Stunden unter Rückfluss. Nach aufeinanderfolgendem Waschen mit Wasser, Natriumhydrogencarbonat-Lösung und nochmals Wasser wird die organische Phase über Natriumsulfat getrocknet und eingeengt. Das Rohprodukt löst man in Diethylether und fällt mit Salpetersäure 34 g des Nitrats von 1-(2,4-Dichlorphenyl)-2- [1-(1,2.4-triazolyl)]- 1-chlorpenten, Fp. 127 °C, die bei Raumtemperatur in 400 ml tert.-Butanol mit 30,2 g Kalium-tert.-butylat 7 Stunden gerührt werden. Dann engt man ein, nimmt in Chloroform auf, wäscht mit Wasser, trocknet nach Abziehen des Lösungsmittels durch Destillation 10 g 1-(2,4-Dichlorphenyl)-2-[1-(1,2,4-Ditriazolyl)]-2-penten. Sdp. 140 bis 144 °C/0,1 mbar.

Analog lassen sich beispielsweise folgende Vinylazole der Formel I herstellen.

| Nr. | R | $X_n$ | Z | Position der Doppelbindung | Fp/Kp[°C] |
|---|---|---|---|---|---|
| 3 | tert.-$C_4H_9$ | 4-Cl | N | a | 78–90 |
| 4 | tert.-$C_4H_9$ | 4-Br | N | a | 133–140 |
| 5 | tert.-$C_{4H}9$ | 4-$CH_3$ | N | a/b | 52–59 |
| 6 | tert.-$C_4H_9$ | H | N | a/b | 110–112/0,2 mbar |
| 7 | tert.-$C_4H_9$ | 4-$C_6H_5$ | N | a | 92/98 |
| 8 | n-$C_3H_7$ | 2-Cl, 4-Cl | N | a/b | 152–156/0,13 mbar |

(Fortsetzung)

| Nr. | R | $X_n$ | Z | Position der Doppelbindung | Fp/Kp[°C] |
|---|---|---|---|---|---|
| 9 | $C_2H_5$ | 2-Cl, 4-Cl | CH | a/b | 170–190/0,4 mbar |
| 10 | tert.-$C_4H_9$ | 2-Cl, 4-Cl | CH | a | 135/0,01 mbar |
| 11 | i-$C_3H_7$ | 2-Cl, 4-Cl | N | a/b | 145–152/0,08 mbar |
| 12 | tert.-$C_4H_9$ | 2-I | N | a/b | Harz |
| 13 | tert.-$C_4H_9$ | 2-Cl, 4-Cl, 6-Cl | N | a/b | Harz |
| 14 | tert.-$C_4H_9$ | 2-I, 6-I | N | a/b | Harz |
| 15 | n-$C_5H_{11}$ | 2-Cl, 4-Cl | N | a/b | Öl |
| 16 | n-$C_6H_{13}$ | 2-Cl, 4-Cl | N | a/b | Öl |
| 17 | 2,4-Dichlorphenyl | 2-Cl, 4-Cl | N | a | Harz |
| 18 | 2,4-Dichlorphenyl | 2-Cl, 4-Cl | CH | a | Harz |
| 19 | 4-Chlorphenyl | 2-Cl, 4-Cl | N | a/b | Harz |

Die neuen Wirkstoffe zeigen eine starke fungitoxische Wirksamkeit gegen phytopathogene Pilze, insbesondere aus den Klassen der Ascomyceten, beispielsweise gegen Erysiphe graminis an Weizen, Erysiphe cichoriacearum an Gurken, Uncinula necator an Reben, Podosphaera leucotricha und Venturia inaequalis an Äpfeln sowie Botrytis cinerea an Reben.

Die neuen Verbindungen können auch im Materialschutz u.a. zur Bekämpfung von Schimmelpilzen, wie Trichoderma viride, von Moderfäulepilzen, wie Chaetomium globosum, oder holzzerstörenden Pilzen, wie Coniophora cerebella und Polystictus versicolor, eingesetzt werden.

Die dabei verwendbaren fungiziden Mittel enthalten 0,1 bis 95 Gew.-%, vorzugsweise 0,5 bis 90 Gew.-% Wirkstoff. Die Aufwandmengen liegen nach Art des gewünschten Effektes zwischen 0,01 und 3 kg oder mehr, vorzugsweise jedoch zwischen 0,01 und 1 kg Wirkstoff/ha.

Die Mittel werden beispielsweise in Form von direkt versprühbaren Lösungen, Pulvern, Suspensionen oder Dispersionen, Emulsionen Öldispersionen, Pasten, Stäubemitteln, Streumitteln, Granulaten durch Versprühen, Vernebeln, Verstäuben, Verstreuen oder Giessen angewendet. Die Anwendungsformen richten sich ganz nach den Verwendungszwecken; sie sollten jn jedem Fall möglichst die feinste Verteilung der Wirkstoffmischungen gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol Paraffin, Tetrahydronaphthalin, alkylierte Naphthaline oder deren Derivate, z.B. Methanol, Ethanol, Propanol, Butanol Chloroform, Tetrachlorkohlenstoff, Cyclohexanol, Cyclohexanon, Chlorbenzol Isophoron, stark polare Lösungsmittel, z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure Naphthalinsulfonsäure Phenolsulfonsäure, Alkyllarylsulfonate, Alkylsulfate, Alkylsulfonate, Alkali- und Erdalkalisalze der Dibutylnaphthalinsulfonsäure, Laurylethersulfat, Fettalkoholsulfate fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalininderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpoly- glykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden

wie Silicagel, Kieselsäuren, Silikate, Talkum, Kaolin, Kalkstein, Kalk, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Beispiele für Formulierungen sind:

I. Man vermischt 90 Gewichtsteile der Verbindung gemäss Beispiel 1 mit 10 Gewichtsteilen N-Methyl-α-pyrollidon und erhält eine Lösung, die zur Anwendung in Form kleinster Tropfen geeignet ist.

II. 10 Gewichtsteile der Verbindung Nr. 5 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanol-amid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

III. 20 Gewichtsteile der Verbindung gemäss Beispiel 3 werden in einer Mischung gelöst, die aus 640 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

IV 20 Gewichtsteile der Verbindung Nr. 3 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

V. 80 Gewichtsteile der Verbindung Nr. 10 werden mit 3 Gewichtsteilen des Natriumsalzes der Diisobutylnaphtalin-α-sulfonsäure, 10 Gewichtsteilen des Natriumsalzes einer Ligninsulfonsäure aus einer Sulfit-Ablauge und 70 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung Nr. 11 werden mit 95 Gewichtsteilen feinteiligem Kaolin innig vermischt. Man erhält auf diese Weise ein Stäubemittel das 5 Gew. % des Wirkstoffs enthält.

VII. 30 Gewichtsteile der Verbindung gemäss Beispiel 2 werden mit einer Mischung aus 92 Gewichtsteilen pulverförmigem Kieselsäuregel und 8 Gewichtsteilen Paraffinöl das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 4 werden mit 10 Teilen Natriumsalz eines Phenolsulfonsäure-harnstoff-formaldehyd-Kondensates 2 Teilen Kieselgel und 48 Teilen Wasser innig vermischt. Man erhält eine stabile wässrige Dispersion. Durch Verdünnen mit Wasser erhält man eine verdünnte wässrige Dispersion.

IX. 20 Teile der Verbindung Nr. 7 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die Wirkstoffe können auch mit anderen bekannten Fungiziden gemischt werden. In vielen Fällen erhält man dabei eine Vergrösserung des fungiziden Wirkungsspektrums; bei einer Anzahl dieser Fungizidmischungen treten synergistische Effekte auf, d.h. die fungizide Wirksamkeit des Kombinationsproduktes ist grösser als die der additiven Wirksamkeit der Einzelkomponenten.

Fungizide, die mit den erfindungsgemässen Vinylazolen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbat,
Tetramethylthiuramdisulfide,
Ammoniak-Komplex von Zink-(N,N-ethylen-bis-dithiocarbamat) und
N, N'-Polyäthylen-bis-(thiocarbamoyl)-disulfid,
Zink-(N, N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2- sec-Butyl- 4,6-dinitrophenyl- 3,3-dimethylacrylat,
2-sec-Butyl-4,6-dinitrophenyl-isopropylcarbonat;

heterocyclische Strukturen, wie
N-Trichlormethylthio-tetrahydrophtalamid,
N-Trichlormethylthio-phthalimid,
2-Heptadecyl-2-imidazolin-acetat,
2,4-Dichlor-6-(o-chloranilino)-s-triazin,
0,0-Diäthyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioantrachinon,
2-Thio-1,3-dithio-(4,5)-b-chinoxalin,
1-(Butylcarbamoyl)-2-benzimidazol-carbaminsäuremethylester,
2-Methoxycarbonylamino-benzimidazol
2-Rhodanmethylthiobenzthiazol,
4-(2-Chlorphenylhydrazono)-3-methyl-5-isoxazolon,

Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2)-benzimidazol,
Piperazin-1,4-diyl-bis-(1-(2,2,2-trichlor-ethyl)-formamid),
2-Thiazolyl-(4)-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol und verschiedene Fungizide, wie
Dodecylguanidinacetat,
3-(3-(3,5-Dimethyl-2-oxycylclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.
5-Nitro-isophtalsäure-diisopropylester,
1-(1', 2',4'-Triazolyl-1')-[1-(4'-chlorphenoxy)]-3,3-dimethylbutan-2-on,
1-(1',2',4'-Triazolyl-1')-[1-(4'chlorphenoxy)]-3,3-dimethylbutan-2-ol,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Die folgenden Beispiele belegen die biologische Wirkung der neuen Verbindungen der Formel I. Vergleichsmittel ist der bekannte fungizide Wirkstoff 1-(1,2,4-Triazol-1-yl)-1-(p-chlorphenacyl) styrol (DE-OS 26 45 617).

Beispiel A

Wirksamkeit gegen Weizenmehltau

Blätter von in Töpfen gewachsenen Weizenkeimlingen der Sorte «Jubilar» werden mit wässrigen Emulsionen aus 80% (Gew-%) Wirkstoff und 20% Emulgiermittel besprüht und nach dem Antrocknen des Spritzbelags mit Oidien (Sporen) des Weizenmehltaus (Erysiphe graminis var. tritici) bestäubt. Die Versuchspflanzen werden anschliessend im Gewächshaus bei Temperaturen zwischen 20 und 22 °C und 75 bis 80 °C relativer Luftfeuchtigkeit aufgestellt. Nach 10 Tagen wird das Ausmass der Mehltaupilzentwicklung ermittelt.

In diesem Test zeigen die Verbindungen Nr. 3, 9 und 10 sowie die Isomeren A und B der Verbindung Nr. 1 gemäss Beispiel 1 eine gute Wirkung.

Wirksamkeit gegen Gurkenmehltau

Blätter von in Töpfen gewachsenen Gurkenkeimlingen werden mit wässrigen Emulsionen einer Mischung aus 80% Wirkstoff und 20% Emulgiermittel besprüht und nach dem Antrocknen des Spritzbelages mit Oidien (Sporen) des Gurkenmehltaus (Erysiphe cichoriacearum) bestäubt. Die Versuchspflanzen werden anschliessend im Gewächshaus bei Temperaturen zwischen 20 und 22 °C und 75 bis 80% relativer Luftfeuchtigkeit aufgestellt. Nach 10 Tagen wird das Ausmass der Mehltaupilzentwicklung ermittelt.

In diesem Test zeigen die Verbindungen 3, 9 und 10 sowie die Isomeren A und B der Verbindung 1 gemäss Beispiel 1 eine bessere Wirkung als das Vergleichsmittel.

Wirksamkeit gegen Botrytis cineara an Paprika

Paprikasämlinge der Sorte «Neusiedler Ideal Elite» werden nachdem sie bis 5 Blätter gut entwickelt haben, mit 0,05 gew-%igen wässrigen Suspensionen, die 80% Wirkstoff und 20% Natriumligninsulfonat in der Trockensubstanz enthalten, tropfnass gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt, um optimale Bedingungen für die Pilzentwicklung zu erhalten. Nach 5 Tagen hat sich die Krankheit auf den unbehandelten Kontrollpflanzen so stark entwickelt, dass die entstandenen Blattnekrosen den überwiegenden Teil der Blätter bedecken.

In diesen Test zeigen die beiden Isomeren A und B der Verbindung 1 gemäss Beispiel 1 und Verbindung Nr. 3 eine sehr gute Wirkung.

## Patentansprüche

1. Vinylazole der Formel

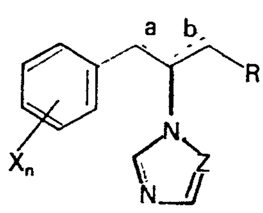

(I)

in der R Alkyl mit 1 bis 7 Kohlenstoffatomen oder gegebenenfalls durch Halogen substituiertes Phenyl,

X Wasserstoff, Halogen, Alkyl mit 1 bis 4 Kohlenstoffatomen oder Phenyl,

Z N oder CH und

n eine ganze Zahl von 1 bis 5 bedeuten und die Doppelverbindung entweder die Position a oder b einnimmt, sowie deren pflanzenverträgliche Säureadditionssalze und Metallkomplexe.

2. Vinylazole der Formel I gemäss Anspruch 1, dadurch gekennzeichnet, dass R Alkyl mit 1 bis 7 Kohlenstoffatomen bedeutet und die Doppelbindung die Position a einnimmt.

3. Vinylazole der Formel I gemäss Anspruch 1, dadurch gekennzeichnet, dass R Alkyl mit 1 bis 7 Kohlenstoffatomen bedeutet und die Doppelbindung die Position b einnimmt.

4. Verfahren zur Herstellung der Vinylazole der Formel I gemäss Anspruch 1, dadurch gekennzeichnet, dass man aus Verbindungen der Formeln

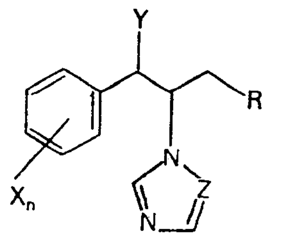

(II) oder

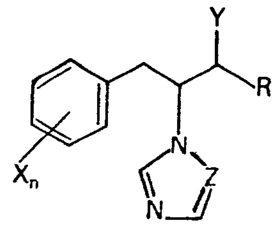

(III)

in denen

R, X, Z und n die im Anspruch 1 angegebenen Bedeutungen haben und Y eine Abgangsgruppe darstellt, HY eliminiert und die so erhaltenen Verbindungen gegebenenfalls in ihre Säureadditionssalze oder Metallkomplexe überführt.

5. Fungizides Mittel, enthaltend inerte Zusatzstoffe und ein Vinylazol der Formel

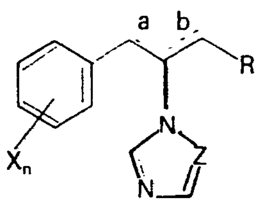

(I)

in der

R Alkyl mit 1 bis 7 Kohlenstoffatomen oder gegebenenfalls durch Halogen substituiertes Phenyl,

X Wasserstoff, Halogen, Alkyl mit 1 bis 4 Kohlenstoffatomen oder Phenyl,

Z N oder CH und

n eine ganze Zahl von 1 bis 5 bedeuten und die Doppelbindung entweder die Position a oder b einnimmt, sowie deren pflanzenverträgliche Säureadditionssalze und Metallkomplexe, als Wirkstoffe.

6. Fungizides Mittel, enthaltend inerte Zusatzstoffe und ein Vinylazol der Formel I gemäss Anspruch 1, in der R Alkyl mit 1 bis 7 Kohlenstoffatomen bedeutet und die Doppelbindung die Position a einnimmt, als Wirkstoff.

7. Fungizides Mittel, enthaltend inerte Zusatzstoffe und ein Vinylazol der Formel I gemäss Anspruch 1 in der R Alkyl mit 1 bis 7 Kohlenstoffatomen bedeutet und die Doppelbindung die Position b einnimmt, als Wirkstoff.

8. Verfahren zur Bekämpfung von Pilzen, dadurch gekennzeichnet, dass man eine fungizid wirksame Menge eines Vinylazols der Formel I gemäss Anspruch 1 auf durch Pilzbefall bedrohte Materialien, Flächen, Pflanzen oder Saatgüter oder auf die Pilze einwirken lässt.

**Revendications**

1. Vinyl-azoles de la formule

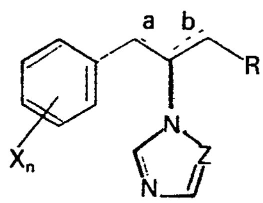

(I)

dans laquelle

R désigne un radical alkyle en $C_1$ à $C_7$ ou un groupe phényle éventuellement halo-substitué,

X représente un atome d'hydrogène ou d'halogène, un radical alkyle en $C_1$ à $C_4$ ou un groupe phényle,

Z = N ou CH et

n est un nombre entier de 1 à 5, la double liaison pouvant occuper la position a ou la position b, ainsi que leurs sels d'addition d'acides et de complexes de métaux phyto-compatibles.

2. Vinyl-azoles de la formule I selon la revendication 1, caractérisés en ce que R désigne un radical alkyle en $C_1$ à $C_7$ et la double liaison occupe la position a.

3. Vinyl-azoles de la formule I selon la revendication 1, caractérisés en ce que R d signe un radical alkyle en $C_1$ à $C_7$ et la double liaison occupe la position b.

4. Procédé de préparation de vinyl-azoles de la formule I selon la revendication 1, caractérisé en ce que, de composés de l'une des formules

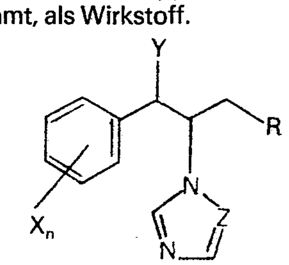

(II) ou

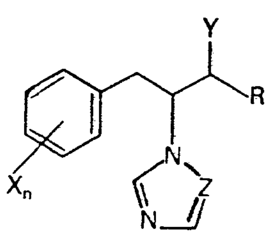

(III)

dans lesquelles R, X, Z et n possèdent les significations définies dans la revendication 1 et Y représente un groupe clivable, l'on élimine le groupe HY et transforme, le cas échéant, les composés obtenus en leurs sels d'addition d'acides ou complexes de métaux.

5. Composition fongicide, contenant des additifs inertes et, en tant que principe actif, un vinylazole de la formule

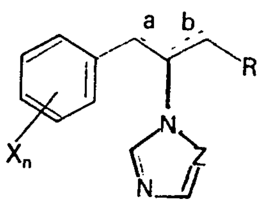

(I)

dans laquelle

R désigne un radical alkyle en $C_1$ à $C_7$ ou un groupe phényle éventuellement halo-substitué,

X représente un atome d'hydrogène ou d'halogène, un radical alkyle en $C_1$ à $C_4$ ou un groupe phényle,

Z = N ou CH et

n est un nombre entier de 1 à 5, la double liaison pouvant occuper la position a ou la position b.

6. Composition fongicide, contenant des additifs inertes et, en tant que principe actif, un vinylazole de la formule I selon la revendication 1, dans laquelle R désigne un radical alkyle en $C_1$ à $C_7$ et la double liaison occupe la position a.

7. Composition fongicide, contenant des additifs inertes et, en tant que principe actif, un vinylazole de la formule I selon la revendication 1, dans laquelle R désigne un radical alkyle en $C_1$ à $C_7$ et la double liaison occupe la position b.

8. Procédé de lutte contre les champignons, caractérisé en ce qu'on fait agir une quantité efficace d'un vinyl-azole de la formule I selon la revendication 1 sur les champignons ou sur les matériaux, surfaces, plantes ou semences à protéger des champignons.

## Claims

1. Vinylazoles of the formula

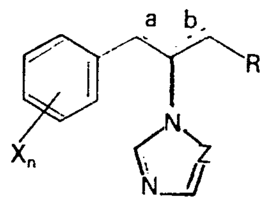

(I)

where R is alkyl of 1 to 7 carbon atoms, phenyl or halogen-substituted phenyl, X is hydrogen, halogen, alkyl of 1 to 4 carbon atoms or phenyl, Z is N or CH, and n is an integer from 1 to 5, and the double bond occupies either position a or position b, and plant-tolerated acid addition salts and metal complexes thereof.

2. Vinylazoles of the formula I as claimed in claim 1, wherein R is alkyl of 1 to 7 carbon atoms and the double bond occupies position a.

3. Vinylazoles of the formula I as claimed in claim 1, wherein R is alkyl of 1 to 7 carbon atoms and the double bond occupies position b.

4. A process for the preparation of vinylazoles of the formula I as claimed in claim 1, wherein HY is eliminated from a compound of the formula

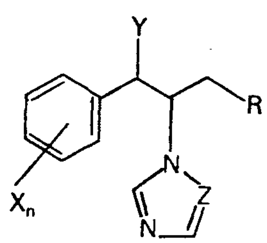

(II) or

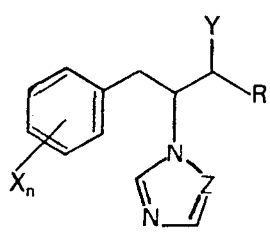

(III)

where R, X, Z and n have the meanings given in claim 1 and Y is a leaving group, and, if desired, the compounds thus obtained are converted into their acid addition salts or metal complexes.

5. A fungicidal agent containing inert additives and a vinylazole of the formula

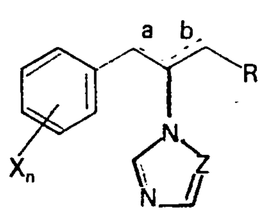

(I)

where R is alkyl of 1 to 7 carbon atoms, phenyl or halogen-substituted phenyl, X is hydrogen, halogen, alkyl of 1 to 4 carbon atoms or phenyl, Z is N or CH, and n is an integer from 1 to 5, and the double bond occupies either position a or position b, as active ingredient.

6. A fungicidal agent containing inert additives and a vinylazole of the formula I as claimed in claim 1, where R is alkyl of 1 to 7 carbon, atoms and the double bond occupies position a, as active ingredient.

7. A fungicidal agent containing inert additives and a vinylazole of the formula I as claimed in

claim 1, where R is alkyl of 1 to 7 carbon atoms and the double bond occupies position b, as active ingredient.

8. A process for combating fungi, wherein a fungicidally effective amount of a vinylazole of the formula I as claimed in claim 1 is allowed to act on the materials, areas, plants or threatened by fungus attack, or on the fungi.